# EUROPEAN PATENT APPLICATION

(11) **EP 4 112 714 A1**
(43) Date of publication of application: **04.01.2023**
(21) Application number: 21199958.6
(22) Date of filing: 29.09.2021
(51) Int. Cl.: C12M 3/04, C12M 1/12, C12M 1/36

(54) **INTEGRATED BIOREACTOR SYSTEM AND PROCESS FOR CONTROL, LEARNING AND MONITORING OF CELL CULTURE USING THE SAME**

(30) Priority: 29.06.2021 PT 2021117309
(71) Applicant: Instituto Politécnico De Leiria, 2411-901 Leiria (PT)
(72) Inventor: RODRIGUES PASCOAL FARIA, PAULA CRISTINA, 2430-028 MARINHA GRANDE (PT); FERNANDES ALVES, NUNO MANUEL, 2430-028 MARINHA GRANDE (PT); MONTEIRO DE MOURA, CARLA SOFIA, 2430-028 MARINHA GRANDE (PT); ALMEIDA MENESES, JOÃO PEDRO, 2430-028 MARINHA GRANDE (PT)
(74) Representative: Monteiro Alves, Inês

(57) **Abstract**

The present invention describes an integrated bioreactor system (1), which comprises an external component (2), an internal component (3) and a support structure (4) which comprises a computational platform for control, learning and optimization of stimuli application.

The present invention further describes a process for the control, learning and monitoring of cell culture using the referred integrated bioreactor system (1), which allows the optimization of the application of different stimuli to cells placed in three-dimensional models (36) located in the internal component (3), to mimic the native conditions of the biological tissue.

This invention solves the problems referring to the need to optimize, control and monitor, in real time, the parameters involved in the stimulation of different cells in tissue engineering applications, thus reducing the time involved in differentiation and / or proliferation process, that are parameterizable for different cell lines.

## Description

### TECHNICAL DOMAIN OF THE INVENTION

The present invention refers to an integrated bioreactor system, which allows the optimization of the application of mechanical, electrical, and magnetic stimuli, which can vary in type and number, in cells placed in three-dimensional models.

The real time monitoring of the stimulation of different cells optimizes, as well, the regeneration process of different types of tissues, such as bone, cartilage, nerve, heart tissue, among others.

### PRIOR ART

Multiple bioreactors have been produced in the sense of recreating specific biologic conditions of the cell type, which it is intended to culture. Although, in a generic manner, they have common objectives, the embodiment thereof leads to specific adaptations to accommodate a suitable flow of nutrients, removal of cellular metabolism by-products, adequate temperature, pH control and, further, cover the application of different types of stimuli, which show themselves to be favorable in some proliferation and / or cell differentiation processes in cell and tissue engineering.

Cell and tissue engineering appears as an alternative to the tissue loss when associating biological substitutes, cells and an extracellular matrix to regenerate the injured tissue or obtain a new tissue and it was initially defined by Skalak and Fox (1988) as the application of the principles and methods of engineering towards the understanding between the structure-function relationships in tissues, whether they are normal or pathological, and the development of biological substitutes to restore, maintain or improve the functions.

In this context, the present invention describes an integrated bioreactor system, which comprises an external component, an internal component and a support structure which comprises a computational platform for control, learning and optimization of the application of the stimulus.

The referred system is capable of carrying out a process of control, learning and monitoring of the cell culture, which allows the optimization of different stimuli in cells placed in three-dimensional models and located in the internal component, to mimic the native conditions of the biological tissue.

The present invention finds some prior art developed and disclosed in literature:
Roberta Visione (2018) developed a modular bioreactor, which combines perfusion and electrical stimulation, further validating the application of these two types of stimuli by means of numerical models.

This bioreactor, in comparison with the invention described herein, has a fixed stimulation geometry, not adaptable, in which the number of electrodes is limited to the space of the central perfusion chamber. The present invention comprises mechanical, electrical, and magnetic stimulation by means of a large number of electrodes or coils which can be activated in an isolated or combined manner, allowing a coordinated electromagnetic stimulation and spatial distribution that is widely controlled.

Although both the system developed in [Roberta Visione 2018] and the integrated bioreactor system of the present invention allow real time monitoring of the cell process by means of a camera/microscope, the bioreactor developed by [Roberta Visione 2018] only allows the use of two electrodes and a spatial distribution that is restricted to this option.

Sara Abasi (2020) developed a static bioreactor system for the application of electromagnetic stimulation, designed by ECSARA, which is able to alternate between the processes of stimulation and monitoring of the cells by means of the AC electric impedance spectroscopy, wherein the delivery of the electric stimulation is determined by resorting to numerical models.

In contrast, as previously described, the present invention further allows the application of mechanical stimulation and a higher capacity of spatially applying the electromagnetic field with more flexibility and possible combinations. In the study developed by [Sara Abasi 2020], numerical models are only used to determine and validate the fields applied, while, in the integrated bioreactor system proposed herein, the numerical models are also used subsequently to monitor, apply, and adapt the fields to the experimental conditions in constant evolution.

Further, resorting to machine learning techniques, this invention further proposes the continuous improvement and integrated optimization of the cell culture process.

Kim-Taek Lim (2019) developed a bioreactor and control system, designed by ABS (acrylonitrile butadiene styrene), which includes the monitoring of the chamber by means of oxygen, carbon dioxide, pressure, temperature sensors; a main control system; a system for renewal and feeding of the culture medium with flow control and a cell culture system, wherein the control of the variables is carried out with the purpose of maintaining the value stipulated at the beginning of the cell culture.

In the present invention, apart from this control, these data are accumulated for machine learning in accordance with the success/failure return of the cell culture. Further, the application of electrical and magnetic stimulation, in an isolated or combined manner, enables a panoply of experimental protocols.

Still in connection with the prior art, we can find the works of Bhaskar Birru (2018) and Isil G. Beskardes (2018) which focus on the creation of perfusion bioreactors for the study of the impact of initial cell culture methods and the intensity of the perfusion flow. These bioreactors consist in modular perfusion chambers; however, they do not comprise integrated monitoring systems, nor do they foresee any application of electric and magnetic fields.

### PROBLEMS OF THE PRIOR ART

The bioreactors described in the studies and publications of the state of the art are incapable of combining, in one sole procedure, the mechanical, electrical, and magnetic stimulation. Further, these do not incorporate control systems for the simultaneous monitoring of these three types of stimulation in a cell process.

The application of mechanical stimuli in a perfusion bioreactor only allows manipulating the flow of the culture medium to obtain a desirable shear stress. The application of electric and magnetic stimulation, in its turn, when carried out as proposed in the present invention, namely, by a large number of electrodes or coils which can be activated in an isolated or combined manner, allows a coordinated electromagnetic stimulation and with widely controlled spatial distribution.

Finally, the state of the art foresees the application of numerical models solely for the prediction and validation of the values of interest, such as current intensity, electric field, and perfusion. In a different manner, in the present invention the learning models guarantee a continuous improvement and the integrated optimization of the cell culture process, thus monitoring and controlling the performance of the bioreactor and adjusting the events in real time.

### SUMMARY OF THE INVENTION

The present invention refers, in a first aspect, to an integrated bioreactor system which comprises:
- an external component (2), comprising an external surface (21) with a plurality of electrodes and/or coils (22), a plurality of orifices (23) and a monitoring window (24);
- an internal component (3), comprising an internal cylindrical surface (31), two lateral surfaces (32), at least one mobile axis (33) with a coaxial entry having at least one shaft (34) and a coaxial outlet having at least one shaft (35); and
- a support chamber (4) comprising a base structure (42), a computer platform (41) and a real time monitoring system with support (43),
   wherein the real time computer platform (41) and the monitoring system with support (43) are configured to allow the control, the learning, and the optimization of the application of stimuli to the extracellular medium and to the three-dimensional model/cell set.

In a second aspect, the invention refers to a process for the control, learning, and monitoring of cell culture, which comprises the steps of:
a) Determining the configurations of the bioreactor according to the desired cell process and type of tissue;
b) Beginning the cell process;
c) Evaluating unobservable parameters of interest;
d) Correlating the configurations of the bioreactor with the unobservable parameters of interest;
e) Compiling the correlated data in a data table for consultation and decision guide on the algorithms of the bioreactor microcontroller;
f) Interpreting and monitoring in real time, by means of control algorithms, all the entry variables of the bioreactor and measurable results; and
g) Integrating the data obtained in (e) and (f) into a data base to carry out machine learning mechanisms, by means of learning algorithms.

### SOLUTION OF THE PROBLEM

The present invention recognizes the existing limitations in the state of the art and solves them by means of the integrated bioreactor system of the present invention.

The integrated bioreactor system of the present invention is configured to integrate the monitoring and isolated or combined application of mechanical, electrical, and magnetic stimuli to one or several three-dimensional models of cell culture.

This system further allows controlling the application and spatial distribution of these stimuli by means of multiple electrodes/coils. Added to that, the ability to apply mechanical stimuli by the application of controlled rotation and translation movements of the set of three-dimensional models, as well as the perfusion stimulation of the same.

The present invention further describes a process for the control, learning, and monitoring of cell culture, wherein the variables of the bioreactor are correlated with a series of parameters collected by a network of sensors and actuators.

The inter-relation of these data with numerical models allows that learning algorithms act continuously in the sense of guaranteeing that the best conditions for a determined cell process are applied, foreseeing the evolution of the multiple parameters, and optimizing the success in obtaining viable and differentiated cell cultures.

### ADVANTAGEOUS EFFECTS OF THE INVENTION

The integrated bioreactor system described in this invention allows the application, isolated or simultaneous, of mechanical, electrical, and magnetic stimuli to mimic the native conditions of the biological tissue.

In this manner, cell proliferation and/or differentiation processes occur in shorter time intervals. Further, the parameters involved in the stimulation of the different cells can be controlled and monitored in real time, allowing the optimization of the experimental process or the interruption thereof in case there are conditions reunited which compromise the tissue regeneration.

### BRIEF DESCRIPTION OF THE FIGURES

With the purpose of providing an understanding of the principles according to the embodiments of the present invention, reference will be made to the embodiments illustrated in the figures and to the terminology used to describe them.

It must also be understood that there is no intention of limiting the scope of the present invention to the content of the figures and that any modifications of the inventive characteristic illustrated herein, such as additional applications of the principles and embodiments shown herein, which would occur normally to a person skilled in the art having this description in hands, are considered as being within the scope of the claimed invention.
Figure 1 - illustrates the integrated bioreactor system of the present invention.
Figure 2 - illustrates the external component of the bioreactor.
Figure 3 - illustrates the internal component of the bioreactor
Figure 4 - illustrates the support structure of the integrated bioreactor system.
Figure 5 - illustrates a flowchart which teaches the control, learning, and monitoring process for the cell culture using the integrated bioreactor system.

### DESCRIPTION OF THE EMBODIMENTS

The present invention describes an integrated bioreactor system (1) which allows the optimization of the application of mechanical, electrical, and magnetic stimuli, which may vary in type and number, in cells placed in three-dimensional models (36) located in the internal component (3) of the integrated bioreactor system (1), to mimic the native conditions of the biological tissue.

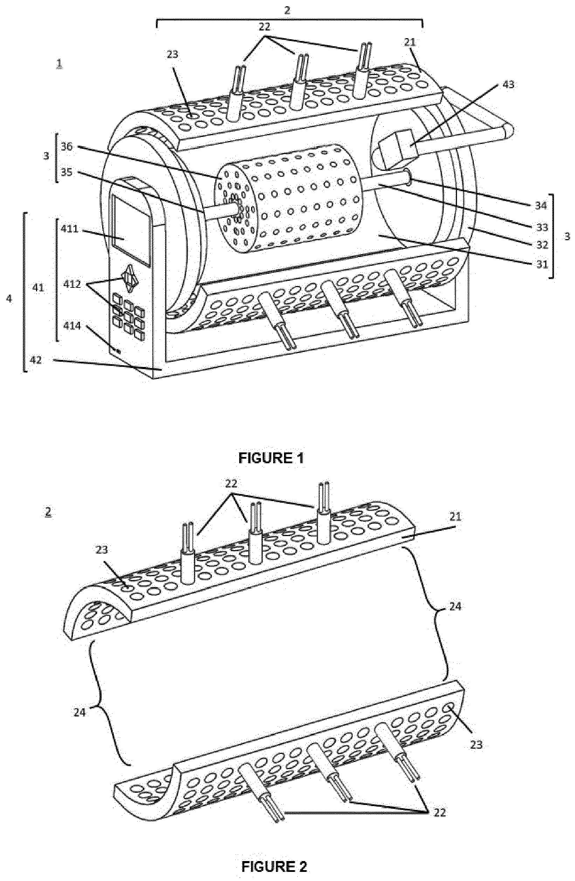

The integrated bioreactor system (1) of the present invention is represented in Figure 1 and comprises:
- an external component (2), comprising an external surface (21) with a plurality of electrodes and/or coils (22), a plurality of orifices (23) and a monitoring window (24);
- an internal component (3), comprising an internal cylindrical surface (31), two lateral surfaces (32), at least one mobile axis (33) having a coaxial entry having at least one shaft (34) and a coaxial outlet having at least one shaft (35); and
- a support chamber (4) comprising a base structure (42), a computer platform (41) and a real time monitoring system with support (43),
   wherein the computer platform (41) and the real time monitoring system with support (43) are configured to allow the control, the learning, and the optimization of the application of stimuli to the extracellular medium and to the three-dimensional model/cell set.

Figure 2 shows the external surface (21) of the external component (2), which is composed by a region for connection and placing of a plurality of electrodes and/or coils (22), a plurality of orifices (23) and a monitoring window region (24).

The plurality of electrodes and/or coils (22) is spatially distributed on the external surface (21), thus allowing the electromagnetic stimulation to be applied in a coordinated form.

The activation of the plurality of electrodes and/or coils (22) occurs on isolated or combined form, wherein the type and the number of stimuli can vary according to the desired cell process, namely, the differentiation and proliferation of the cells of interest.

In one preferred embodiment of the present invention, the external surface (21) comprises a plurality of orifices (23) which allows the perfusion of a suitable fluid medium for the desired stimulation. The number of orifices (23) depends on the size of the integrated bioreactor system (1), which, in turn, is determined in accordance with the experimental protocol to be carried out, being, therefore, customizable, and adaptable.

Further, the external surface (21) is made from electric insulating material, to be safer to the user during handling.

The monitoring window (24) of the external component (2) is also customizable and adaptable to the optical equipment used. In this manner, it presents a variable dimension and works as an optical access for use of the real time monitoring system with support (43).

The referred monitoring window (24) allows the continuous and non-invasive evaluation of the cell culture in the three-dimensional models (36).

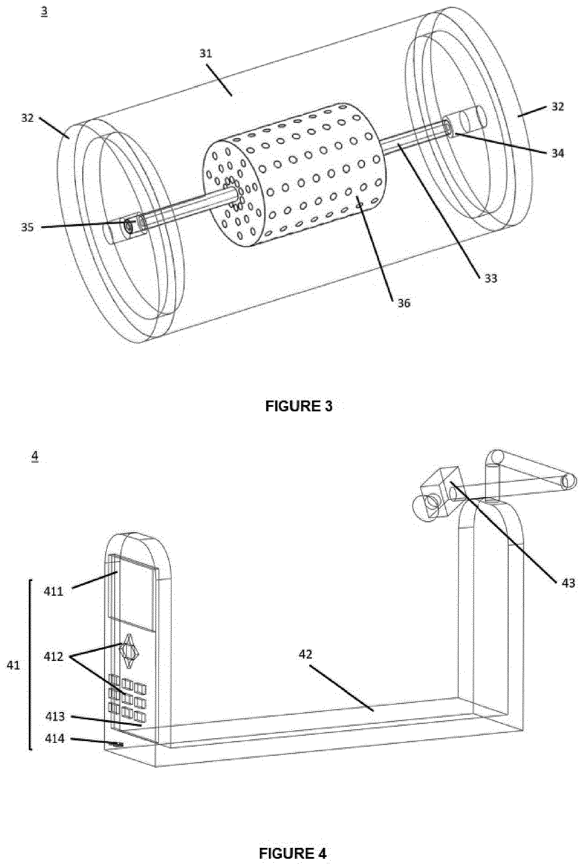

As can be observed in Figure 3, the internal component (3) of the integrated bioreactor system (1) of the present invention comprises an internal cylindrical surface (31) and two lateral surfaces (32), which, together, form a cell culture region, wherein the at least one three-dimensional model (36) will be allocated.

The referred internal cylindrical surface (31) interfaces with the external surface (21) of the external component (2) and, in a preferred embodiment, is transparent and made from non-impeding/reactive material to the passage of the stimuli originating from the plurality of electrodes and/or coils (22) distributed on the external surface (21).

The internal component (3) further comprises at least one mobile axis (33), which is rotary and responsible for the application of the multidirectional mechanical stimulation in the three-dimensional models (36).

The referred mechanical stimuli are due to controlled rotation and translation movements of the at least one three-dimensional model (36) in the mobile axis (33), as well as the stimulation of the perfusion of the same with a culture medium that is suitable to the desired cell process.

In one preferred embodiment of the invention, at least one mobile axis (33) is tubular and presents a central perforation that allows the feeding of the culture medium in the cell culture region. This cell culture region grants watertightness and can further include rotating blades (optional) on the internal cylindrical surface (31) and on the two lateral surfaces (32) for a better perfusion of the culture medium.

The culture medium is inserted in the cell culture region by means of the coaxial entry having at least one shaft (34) and drained by the coaxial outlet having at least one shaft (35). The coaxial outlet (35) will be used or not, according to whether it is intended to renew or not the cell medium during the cell process.

Both the external (2) and internal (3) components comprise adaptable volume for the incorporation of one or multiple three-dimensional models (36) and one or multiple mobile axis (33).

The support structure (4) sustains the external (2) and internal (3) components by means of the lateral surfaces (32) and is comprised of elements that grant mechanical resistance to the device.

The support structure (4), which is represented in Figure 4, includes all the electronics and remaining components that are necessary for the creation of the computer control, learning, and optimization platform (41) for application of stimuli.

The computer platform (41) consists of a screen (411), command and function buttons (412), a microcontroller and/or optimization, learning, and control processor (413) and communication and expansion ports (414), which are incorporated into a base structure (42). A real time monitoring system (42) is also available and allows accompanying the regeneration process by a video/thermographic camera.

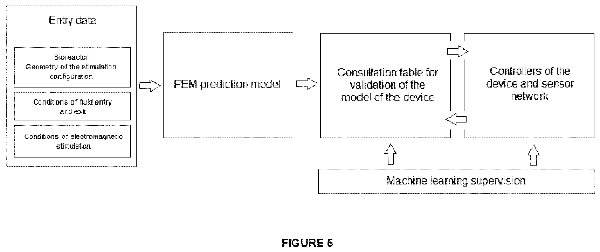

This platform (41) allows executing a control, learning, and monitoring process in real time of the parameters involved in the stimulation of different cells, which can further be optimized by means of numerical modeling and learning techniques, such as shown in the flowchart of figure 5.

The referred process comprises the steps of:
a) Determining the configurations of the bioreactor according to the desired cell process and type of tissue;
b) Beginning the cell process;
c) Evaluating unobservable parameters of interest;
d) Correlating the configurations of the bioreactor with the unobservable parameters of interest;
e) Compiling the correlated data in a data table for consultation and decision guide of the algorithms of the bioreactor microcontroller;
f) Interpreting and monitoring in real time, by means of control algorithms, all the entry variables of the bioreactor and measurable results; and
g) Integrating the data obtained in (e) and (f) into a data base to carry out machine learning mechanisms, by means of learning algorithms.

In step (a), the configurations of the bioreactor (type and number of stimuli, duration, intensity, frequency, and form of signal) are determined according to the desired cell process and the type of tissue present in the three-dimensional model (bone, cartilage, heart tissue, among others). Next, the cell process is begun ((step b))

In step (c), some unobservable parameters of interest are evaluated. In one preferred embodiment of this invention, the parameters of interest are parameters of a physicochemical nature that are relevant for the cell process, being selected from the group consisting of electromagnetism, fluid dynamics, heat transfer and chemical diffusion. This evaluation is carried out by means of a network of sensors and actuators, not shown in the figures.

In step (d), the configurations of the bioreactor and the unobservable parameters of interest are correlated and, subsequently (in step (e)), compiled in a table. The data of this table will be considered for the decision guide on control algorithms of the microcontroller of the bioreactor, which will, in step (f), interpret and monitor in real time all the entry variables of the bioreactor and measurable results, allowing to act constantly on the evolution of the parameters based on the tables obtained in step (e).

The continuous process records, as well as the results of the cell culture, will, finally in step (g) be integrated into a database to execute machine learning mechanisms.

From that on, the learning algorithms act continuously in the sense of guaranteeing that optimum conditions are applied, foreseeing the evolution of the multiple parameters, and optimizing the success in obtaining the viable and differentiated cell cultures.

In this manner, the integrated bioreactor system allows, by means of the control, learning, and monitoring process in real time of the parameters involved in the stimulation of different cell types, to determine the best stimulation protocol and use and monitor the application of the referred protocol, whereby the system may be interrupted in case the values observed in the cell culture region compromise the desired cell process.

The subject matter described above is provided as an illustration of the present invention and, therefore, cannot be interpreted so as to limit it. The terminology used herein with the purpose of describing preferred embodiments of the present invention, must not be interpreted to limit the invention to the same.

As used in the specification, the definite and indefinite articles, in their singular form, aim at the interpretation of also including the plural forms, unless the context of the description indicates, explicitly, the contrary.

The indefinite article "one" must be interpreted generally as "one or more", unless the sense of a singular embodiment is clearly defined in a specific situation.

It will be understood that the expressions "comprise" and "include", when used in this description, specify the presence of the characteristics, the elements, the components, the steps, and the related operations, however, they do not exclude the possibility of other characteristics, elements, components, steps, and operations that can also be contemplated.

As used throughout this patent application, the expression "or" is used in the inclusive sense, unless the exclusive sense is clearly defined in a specific situation. In this context, a sentence of the type "X uses A or B" must be interpreted as including all the pertinent inclusive combinations, for example "X uses A", "X uses B" and "X uses A and B".

All the alterations, providing that they do not modify the essential characteristics of the claims that follow, must be considered as being within the scope of protection of the present invention.

### LIST OF REFERENCE INDICATIONS

1) Integrated bioreactor system
2) External component of bioreactor
   21) External surface
   22) Electrodes and/or coils
   23) Orifices
   24) Monitoring Window
3) Internal component of bioreactor
   31) Internal cylindrical surface
   32) Lateral surfaces
   33) Mobile axis or spool(s)
   34) Axial entry with spool(s)
   35) Axial outlet with spool(s)
   36) Three-dimensional model(s)
4) Support structure
   41) Optimization and control platform
   411) Screen
   412) Command and function buttons
   413) Microcontroller and/or optimization, learning and control processor
   414) Communication and expansion ports
42) Base structure
43) Real time monitoring system with support (video/thermographic camera)

### LIST OF CITATIONS

Follows the list of citations:

### NON-PATENTARY LITERATURE

R. Skalak, "Tissue engineering", Proceedings of the 15th Annual International Conference of the IEEE Engineering in Medicine and Biology Society, San Diego, CA, USA, 1993, pp. 1112 - 1113, doi: 10.1109/IEMBS.1993.979045.
Visone, Roberta, Giuseppe Talo, Silvia Lopa, Marco Rasponi, and Matteo Moretti, "Enhancing All-in-One Bioreactors by Combining Interstitial Perfusion, Electrical Stimulation, on-Line Monitoring and Testing within a Single Chamber for Cardiac Constructs". 2018, Scientific Reports 8 (1): 16944.
Abasi, Sara, John R. Aggas, Naren Venkatesh, Iris G. Vallavanatt, and Anthony Guiseppi-Elie, "Design, Fabrication and Testing of an Electrical Cell Stimulation and Recording Apparatus (ECSARA) for Cells in Electroculture". 2020, Biosensors & Bioelectronics 147 (January): 111793.
Lim, Ki-Taek, Dinesh K. Patel, Hoon Seonwoo, Jangho Kim, and Jong Hoon Chung, "A Fully Automated Bioreactor System for Precise Control of Stem Cell Proliferation and Differentiation". 2019, Biochemical Engineering Journal 150 (June): 107258.
Birru, Bhaskar, Naveen Kumar Mekala, and Sreenivasa Rao Parcha, "WITHDRAWN Highlights - Improved Osteogenic Differentiation of Umbilical Cord Blood MSCs Using Custom Made Perfusion Bioreactor". 2019, Biomedical Journal, March. https://doi.org/10.1016/j.bj.2018.11.003.
Be 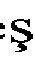 karde 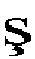 , I 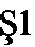 l G., Gizem Ayd 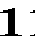 n, 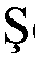 eyma Bekta 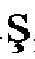 , Alper Cengiz, and Menem 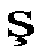 e Gümü 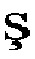 derelio 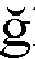 lu, "A Systematic Study for Optimal Cell Seeding and Culture Conditions in a Perfusion Mode Bone-Tissue Bioreactor". 2018, Biochemical Engineering Journal 132 (April): 100 - 111.

## Claims

1. Integrated bioreactor system (1) **characterized by** comprising:
- an external component (2), comprising an external surface (21) with a plurality of electrodes and/or coils (22), a plurality of orifices (23) and a monitoring window (24);
- an internal component (3), comprising an internal cylindrical surface (31), two lateral surfaces (32), at least one mobile axis (33) having a coaxial entry having at least one shaft (34) and a coaxial outlet having at least one shaft (35); and
- a support chamber (4) comprising a base structure (42), a computer platform (41) and a real time monitoring system with support (43),
wherein the computer platform (41) and the real time monitoring system with support (43) are configured to allow the control, the learning, and the optimization of the application of stimuli to the extracellular medium and to the three-dimensional model/cell set.

2. Integrated bioreactor system (1), according to claim 1, **characterized by** the external surface (21) of the external component (2) comprising a plurality of electrodes and/or coils (22) spatially distributed and configured to promote the electrical and magnetic stimulation of the three-dimensional model (36).

3. Integrated bioreactor system (1), according to claim 2, **characterized by** the plurality of electrodes and/or coils (22) being activated in isolated or combined form.

4. Integrated bioreactor system (1), according to any one of the preceding claims, **characterized by** the external surface (21) comprising a plurality of orifices (23) and being made from electric insulating material.

5. Integrated bioreactor system (1), according to any one of the preceding claims, **characterized by** the internal component (3) comprising an internal cylindrical surface (31) and two lateral surfaces (32) to form a cell culture region.

6. Integrated bioreactor system (1), according to any one of the preceding claims, **characterized by** the internal cylindrical surface (31) interfacing with the external surface (21) of the external component (2).

7. Integrated bioreactor system (1), according to any one of the preceding claims, **characterized by** the internal component (3) comprising at least one rotary mobile axis (33) and being configured to promote the mechanical stimulation of the three-dimensional model (36) by means of controlled rotation and translation movements and perfusion of the same with a culture medium.

8. Integrated bioreactor system (1), according to any one of the preceding claims, **characterized by** the at least one mobile axis (33) being tubular and presenting a central perforation that is configured to allow the feeding of the culture medium in the cell culture region.

9. Integrated bioreactor system (1), according to any one of the preceding claims, **characterized by** the internal cylindrical surface (31) and the two lateral surfaces (32) optionally including rotating blades.

10. Integrated bioreactor system (1), according to any one of the preceding claims, **characterized by** both the external (2) and internal (3) components presenting adaptable volume and the monitoring window (24) of the external component (2) being customizable and adaptable to the optical equipment used.

11. Integrated bioreactor system (1), according to any one of the preceding claims, **characterized by** the support structure (4) sustaining the external (2) and internal (3) components by means of the lateral surfaces (32).

12. Integrated bioreactor system (1), according to any one of the preceding claims, **characterized by** the computer platform (41) consisting of a screen (411), command and function buttons (412), a microcontroller and/or optimization, learning, and control processor (413) and communication and expansion ports (414), which are incorporated into a base structure (42).

13. Process for the control, learning and monitoring of cell culture, executed by the integrated bioreactor system (1) as defined in any one of claims 1 to 12, **characterized by** comprising the steps of:
a) Determining the configurations of the bioreactor according to the desired cell process and type of tissue;
b) Beginning the cell process;
c) Evaluating unobservable parameters of interest;
d) Correlating the configurations of the bioreactor with the unobservable parameters of interest;
e) Compiling the correlated data in a data table for consultation and decision guide on the algorithms of the bioreactor microcontroller;
f) Interpreting and monitoring in real time, by means of control algorithms, all the entry variables of the bioreactor and measurable results; and
g) Integrating the data obtained in (e) and (f) into a data base to carry out machine learning mechanisms, by means of learning algorithms.

14. Process, according to claim 13, **characterized by**, in step (a), the configurations of the bioreactor being determined in accordance with the desired cell process and type of tissue present in the three-dimensional model.

15. Process, according to any one of claims 13 or 14, **characterized by**, in step (c), the evaluation of the unobservable parameters of interest being carried out by means of a network of sensors and actuators.

16. Process, according to claim 15, **characterized by** the parameters of interest being parameters of a physicochemical nature that are relevant for the cell process, being selected from the group consisting of electromagnetism, fluid dynamics, heat transfer and chemical diffusion.

17. Process, according to any one of claims 13 to 15, **characterized by**, in step (f), the data compiled in the table being considered as the decision guide on the control algorithms of the microcontroller of the integrated bioreactor system (1).

18. Process, according to any one of claims 13 to 16, **characterized by**, in step (g), the learning algorithms acting continuously and being configured to determine the optimum stimulation protocol for obtaining viable and differentiated cell cultures.
